**Europäisches Patentamt**

**European Patent Office**

**Office européen des brevets**

(11) Publication number : **0 603 031 A2**

# (12) EUROPEAN PATENT APPLICATION

(21) Application number : **93402977.8**

(22) Date of filing : **09.12.93**

(51) Int. Cl.⁵ : **C07K 15/04,** C12N 1/20,
// A61K37/02 , (C12N1/20,
C12R1:465)

(30) Priority : **14.12.92 US 990167**

(43) Date of publication of application :
**22.06.94 Bulletin 94/25**

(84) Designated Contracting States :
**AT BE CH DE DK ES FR GB GR IE IT LI LU MC
NL PT SE**

(71) Applicant : **Bristol-Myers Squibb Company**
**345 Park Avenue**
**New York, N.Y. 10154 (US)**

(72) Inventor : **Nishio, Maki**
**2-10-2, Nishikamata,**
**Ohta-ku**
**Tokyo (JP)**
Inventor : **Kawano, Kimio**
**967-1-315, Kashiwagaya**
**Ebina (JP)**
Inventor : **Yamamoto, Satoshi**
**2-14-404, Sakonyama,**
**Asahi-ku**
**Yokohama (JP)**

(74) Representative : **Durand, Yves Armand Louis**
**et al**
**CABINET WEINSTEIN**
**20, Avenue de Friedland**
**F-75008 Paris (FR)**

(54) **Antiviral antibiotic BU-4724V and preparation thereof.**

(57)    There is provided a new antiviral antibiotic designated herein as BU-4724V which is produced by fermentation of a BU-4724V-producing strain of a new microorganism, Streptomyces sp. ATCC 55290. Antibiotic BU-4724V is recovered and purified from the fermentation broth by use of extraction and chromatography techniques. BU-4724V has been found to have some antibacterial activity and inhibits the growth of HIV and HSV viruses.

EP 0 603 031 A2

## BACKGROUND OF THE INVENTION

The present invention relates to a novel antiviral antibiotic designated BU-4724V having antibacterial and antiviral activities that is useful for treating bacterial and viral infections. It also relates to a process of producing the novel antibiotic by fermentation of a new microorganism Streptomyces sp. and to a pharmaceutical composition thereof.

## SUMMARY OF THE INVENTION

This invention relates to a new antiviral antibiotic designated herein as BU-4724V, to its preparation by fermentation of a new microorganism, Streptomyces sp. ATCC 55290, or a mutant thereof, in an aqueous fermentation culture nutrient medium containing assimilable sources of nitrogen and carbon under submerged aerobic conditions until a substantial amount of the antiviral antibiotic designated BU-4724V is produced by the organism in the fermentation culture nutrient medium, and to its recovery from the fermentation medium. This invention also relates to the pharmaceutical compositions containing the new antiviral antibiotic and methods for using said antiviral antibiotic as an antimicrobial and antiviral agent.

## DESCRIPTION OF THE DRAWINGS

FIG. 1 shows the infrared (IR) absorption spectrum of BU-4724V (KBr, pellet).
FIG. 2 shows the proton magnetic resonance ($^1$H-NMR) spectrum of BU-4724V in $CD_3OD$ (400 MHz).

## DETAILED DESCRIPTION OF THE INVENTION

The present invention relates to a new antiviral antibiotic, designated BU-4724V, which is produced by fermenting a BU-4724V-producing strain of Streptomyces sp. ATCC 55290.

In another aspect, this invention provides a pharmaceutical composition of BU-4724V.

In yet another aspect, this invention provides a process for producing the novel antiviral antibiotic BU-4724V.

In still another aspect, this invention provides a method for inhibiting the growth of viruses in human blood cells comprising contacting such viruses with a growth-inhibitory effective amount of the novel antiviral antibiotic, BU-4724V.

In still yet another aspect, this invention provides a novel microorganism Streptomyces sp. ATCC 55290.

The novel antiviral antibiotic BU-4724V is obtained by fermentation of a new microorganism classified as a species of the genus Streptomyces, accumulating BU-4724V produced by said microorganism and collecting the antiviral antibiotic BU-4724V from the culture broth. A preferred BU-4724V-producing microorganism is the Streptomyces sp. Strain AA6532 isolated from a soil sample collected at Shinjuku, Tokyo, Japan. Based on the taxonomic descriptions of this genus and the comparative studies to the relevant species, Strain AA6532 was classified as Streptomyces sp. and was deposited in the American Type Culture Collection, Rockville, MD, under the accession number ATCC 55290. The permanency of the deposit of this culture at the American Type Culture Collection at Rockville, MD, and ready accessibility thereto by the public are afforded throughout the effective life of the patent in the event the patent is granted. Access to the culture is available during pendency of the application under 37 C.F.R. 1.14 and 35 U.S.C. 112. All restrictions on the availability to the public of the culture ATCC 55290 deposited will be irrevocably removed upon granting of the patent.

## THE MICROORGANISM

The following is a general description of the preferred microorganism producing the antiviral antibiotic BU-4724V.

### Taxonomy

The microorganism producing BU-4724V, Strain AA6532 was isolated from a soil sample collected at Shinjuku, Tokyo, Japan.

The taxonomic studies were carried out mostly according to the procedure by the International Streptomyces Project (ISP) using media recommended by E.B. Shirling, et al, in Intern. J. Syst. Bacteriol, 16, 313-340 (1966), S.A. Waksman in The Actinomycetes, II, pp. 328-334, Williams and Wilkins Co., Baltimore (1961) and T. Arai, in In Culture Media for Actinomycetes, The Society of Actinomycetes Japan (1975). Incubation was carried out at 28°C for 21 days. Morphological observations were made with both light and electron microscopes on the cultures grown at 28°C for 21 days on oatmeal agar (ISP-3). Color assignment was made using the Manual of Color Names (Japan Color Enterprize Co., Ltd,, 1987).

Cell wall analysis was performed by the methods of B. Becker, et al. in Appl. Microbiol. 12: 421-423 (1964) and B. Becker, et al. in Appl. Microbiol. 13: 236-243 (1965). Phospholipid and mycolate compositions were determined by the methods of M. P. Lechevalier, in J . Lab. Clin. Med. 71: 934-944 (1968), M.P. Lechevalier, et al. in Biochem. Syst. Ecol., 5: 249-260 (1977) and D.E. Minnikin, et al. in J. Gen. Microbiol. 88: 200-204 (1975), respectively. Menaquinone was analyzed by the procedures of M.D. Collins, et al. J. Appl. Bacteriol. 48: 277-282 (1980). Fatty acid type was determined by the methods of K. Suzuki, et al. in Int. J. Syst. Bacteriol. 33: 188-200 (1983).

Temperature range for growth was determined on yeast starch agar using a temperature gradient incubator TN-3 (Toyo Kagaku Snagyo Co., Ltd.).

## Morphology

Strain AA6532 formed olive gray to brownish gray aerial mycelia on various agar media. On observation with a light microscope, the aerial mycelia showed the presence of tight-spiral (Section: Spirales). Scanning electron micrographs indicated that more than 20 cylindrical (0.6 x 1.2 $\mu$m) spores were formed in chains and the surface of the spores were smooth.

## Cultural Characteristics

The appearance of strain AA6532 on 14 agar media is shown in Table 1. The strain grew well and sporulated on both synthetic and organic media. Mature aerial mycelia were generally powdery. The color of aerial mass was olive gray to brownish gray (Gray-color series). The color of vegetative mycelia and reverse side of colony was yellowish brown. No diffusible pigment was produced in various agar media.

The physiological characteristics and the utilization of carbon sources are shown in Tables 2 and 3, respectively. Strain AA6532 grew at temperatures ranging from 10 to 40°C with an optimum range of 25 to 30°C.

## Chemotaxonomy

Analysis of whole cell hydrolysates of Strain AA6532 showed the presence of LL-diaminopimeric acid, galactose, glucose, ribose and mannose. Accordingly, the cell wall of this strain is classified type I and the sugar type is not characterized. Phosphatidylcholine and mycolic acid were not detected. Analysis of the menaquinone composition showed 50% MK-9($H_8$), 31% MK-9($H_6$), 9% MK-9($H_4$), 6% MK-9($H_{10}$), 3% NK-9($H_2$) and 1% MK-9($H_{12}$). Fatty acid type is branched chain 2C (Table 4).

From the taxonomic properties described above, Strain AA6532 is considered as belonging to the genus Streptomyces Waksman and Henrici (1943). Thus, Strain AA6532 is identified as a species of Streptomyces.

**TABLE 1**

| Cultural Characteristics of Strain AA6532 | | | | |
|---|---|---|---|---|
| Medium | Growth | Reverse | Aerial Mycelium | Soluble Pigment |
| Sucrose-nitrate agar (Waksman med. No. 1) | Yellowish white (393) | Pale yellow (127) | Olive gray (410) powdery | None |
| Glycerol nitrate agar | Greenish gray (412) | Olive (167) | Light gray (397) powdery, good | None |
| Glucose asparagine agar (Waksman med. No. 2) | Beige gray (401) | Beige gray (401) | Olive gray (401) powdery, good | None |
| Yeast extract-malt extract agar (ISP med. No. 2) | Dull yellow (150) | Dull yellow (152) | Olive gray (410) powdery, good | None |
| Oatmeal agar (ISP med. No. 3) | Yellowish white (393) | Yellowish white (393) | Brownish gray (409) powdery, good | None |
| Inorganic salts-starch agar (ISP med. No. 4) | Grayish yellow (157) | Grayish yellow (157) | Grayish olive (168) powdery, good | None |
| Glycerol asparagine agar (ISP med. No.5) | Yellowish gray (402) ~ Brownish black (428) | Brownish black (428) | Brownish gray (409) powdery, good | None |
| Tyrosine agar (ISP med. No. 7) | Dark gray (415) | Brownish black (428) | Grayish yellow (156) powdery, good | None |
| Nutrient agar (Waksman med. No. 14) | Dull yellow (150) | Dull yellow (150) | White (388) powdery, good | None |
| Yeast starch agar | Grayish yellow (157) | Grayish yellow (157) | Olive gray (410) powdery, good | None |
| Gauze's agar | Yellowish gray (402) | Yellowish gray (402) | Olive gray (410) powdery, good | None |
| Oatmeal-yeast extract agar | Beige gray (401) | Beige gray (401) | Brownish gray (409) powdery, good | None |
| Bennett's agar (Waksman med. No. 30) | Grayish yellow (155) | Dull yellow (152) | Olive gray (410) powdery, good | None |
| Maltose-Bennett's agar | Brownish olive (163) | Brownish olive (163) | Brownish gray (409) powdery, good | None |

**TABLE 2**

| Physiological Characteristics of Strain AA6532 | |
|---|---|
| Test | Results |
| Starch hydrolysis (On ISP med. No. 4) | Positive |
| Nitrate reduction (Difco, nitrate broth) | Negative |
| Milk (Difco, 10% skimmed milk) | |
| Coagulation | Negative |
| Preptonization | Positive |
| Cellulose decomposition (sucrose nitrate solution with a strip of paper as the sole carbon source) | Negative (Growth: good) |
| Gelatin liquefaction | |
| On plain gelatin | Negative |
| On glucose peptone gelatin | Negative |
| Melanin formation (On ISP med. No. 7) | Positive (weak) |
| Temperature range for growth (°C) | 10 - 40 |
| Optimum temperature (°C) (On Yeast starch agar) | 25 - 35 |
| pH range for growth | 5 - 8 |
| Optimum pH (On trypticase soy broth, BBL) | 6 - 7 |

**TABLE 3**

Utilization of Carbon Sources by Strain AA6532

| Carbon Source | Growth |
|---|---|
| D-Glucose | ++ |
| L-Arabinose | + |
| D-Xylose | ++ |
| Inositol | ++ |
| Mannitol | ++ |
| D-Fructose | ++ |
| L-Rhamnose | ++ |
| Sucrose | ++ |
| Raffinose | ++ |

+: Weak positive
++: Strong positive
Medium: ISP med. No. 9, after incubation at 28°C for 21 days

**TABLE 4**

Fatty Acid Composition of Strain AA6532

Fatty Acid Composition (%)

| Straight Chain | | | | | Branched Chain | | | | | |
|---|---|---|---|---|---|---|---|---|---|---|
| 14:0 | 15:0 | 16:0 | 17:0 | | $i$-14 | $i$-15 | $i$-16 | $i$-17 | a-15 | a-17 |
| 1 | 3 | 14 | 2 | | 5 | 11 | 15 | 10 | 21 | 12 |

| Unsaturated Chain | | | | |
|---|---|---|---|---|
| $i$-16:1 | 16:1$^9$ | $i$-17:1 | a-17:1 | 17:0$^\Delta$ |
| 1 | 1 | 2 | 1 | 1 |

## PREPARATION, ISOLATION, AND PURIFICATION OF BU-4724V

The process for producing the antiviral antibiotic BU-4724V, according to the present invention, comprises the steps of:

(a) cultivating Streptomyces sp. ATCC 55290 in an aqueous fermentation culture nutrient medium containing assimilable sources of nitrogen and carbon under submerged aerobic conditions until a substantial amount of BU-4724V is produced in the fermentation culture medium;

(b) extracting the antibiotic component from the whole fermentation broth with an organic solvent;

(c) adsorbing the antibiotic component contained in the extract from Step (b) on selective chromatography columns;

(d) separating the antibiotic component adsorbed to the column in Step (c) by selective gradient elution techniques; and

(e) recovering from the eluate from Step (d) the antiviral antibiotic BU-4724V by at least one conventional adsorption technique and crystallization or lyophilization technique.

The nutrient medium should contain an appropriate assimilable carbon source for use in the aqueous fermentation culture medium and may be a carbohydrate such as, for example, glucose, ribose, galactose, fructose, mannose, sucrose, lactose, soluble starch, and glycerol to name a few. The assimilable nitrogen source for use in the aqueous fermentation culture medium may be any one of such conventionally known sources, including fish meal, soybean meal, corn steep liquor, peptones, meat extract, peanut flour, yeast extract, and ammonium salts to name but a few. Inorganic salts, such as sodium chloride, potassium chloride, magnesium sulfate, calcium carbonate, phosphates, and the like, may be added if desired. In addition, trace elements, such as copper, manganese, iron, zinc, and the like, may be added if desired or they may be supplied as minor or trace impurities of other constituents in the fermentation media.

Production of the antiviral antibiotic BU-4724V may be effected at any temperature conducive to satisfactory growth of the producing organism such as 10°-40°C and is most conveniently carried out at a temperature of about 25°-32°C. A neutral or nearly neutral initial pH, for example, pH about 6-8, is preferably employed in the fermentation media, and production of the antiviral antibiotic by fermentation is generally carried out for a period of about 2-10 days. Ordinarily, optimum production is achieved in about 4-7 days. For the preparation of relatively small amounts of the antiviral antibiotic, shake flasks and surface culture can be employed whereas for relatively large amounts submerged aerobic culture in sterile fermentation tanks are preferred. When tank fermentation is to be carried out, it is desirable to produce a vegetative inoculum in a nutrient broth by inoculating the broth culture with a spore from the organism and, when a young active vegetative inoculum has been obtained, transferring the inoculum aseptically to the fermentation tank medium. Aeration in tanks and bottles may be provided by forcing sterile air through or onto the surface of the fermentation medium. Further agitation of the medium may be provided by a mechanical impeller and an antifoaming agent such as is conventional in the art, e.g., lard oil may be added as needed.

The production of the antiviral antibiotic BU-4724V in the fermentation medium may be followed readily during the course of the fermentation by syncytium formation inhibition assay.

After optimum fermentation broth potency has been obtained (as determined by the above-described assay method), the whole fermentation broth may be extracted with an organic solvent which contains antibiotic activity. The component having antibiotic activity can be recovered from the organic extract by employing conventional adsorption techniques.

In one preferred embodiment, the fermentation broth is extracted with an alkanol solvent, preferably n-butanol, and the solid produced after concentration was dissolved in alkanol, preferably methanol, and applied to a column packed with Diaion HP-20 resin (Mitsubishi Chemical Industries), and the resin is washed with 30% aqueous methanol and then eluted with 80% aqueous methanol. The eluate fractions having antibiotic activity are combined and concentrated *in vacuo.* The resulting crude solid is then applied to a column packed with YMC GEL ODS A60 (YMC Co.) and the column is eluted with acetonitrile - 0.15% $KH_2PO_4$ (4:6) at pH 3.5. The resulting antibiotic activity - containing eluate is concentrated *in vacuo* and then extracted with n-butanol. The semi-pure solid from the concentrated extract was further purified on a column packed with Sephadex LH-20 resin (Pharmacia Fine Chemicals Inc.) and the column eluted with methanol to produce a white solid of pure antiviral antibiotic BU-4724V.

PHYSICO-CHEMICAL CHARACTERIZATION OF BU-4724V

The antiviral antibiotic BU-4724V was isolated as a white amorphous powder. It is soluble in dimethyl sulfoxide, dimethylformamide, methanol and ethanol, and slightly soluble in alkaline water, but insoluble in water, ethyl acetate, chloroform, benzene, n-hexane and petroleum ether. It gave a positive color reaction with sulfuric acid but a negative color reaction with ninhydrin reagent.

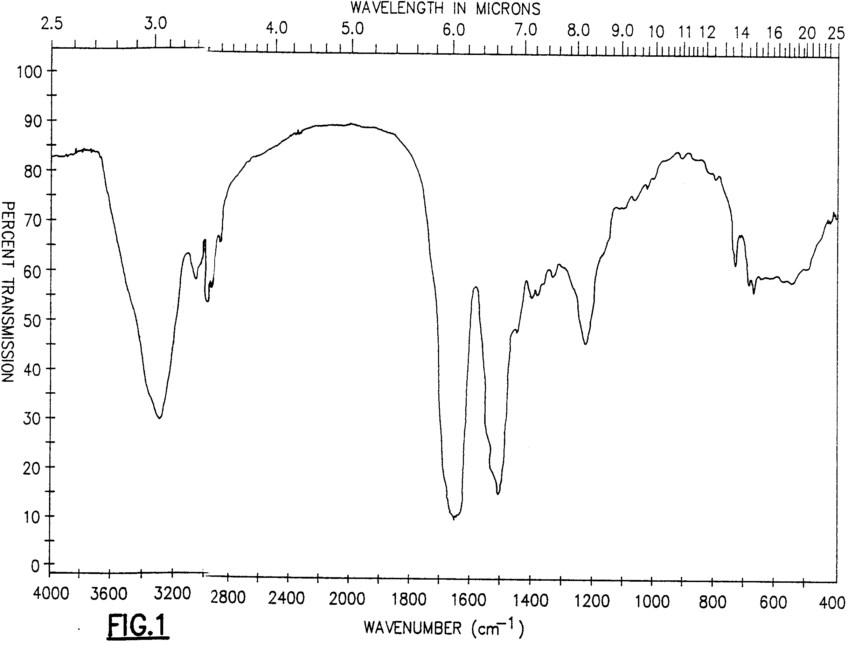

The physico-chemical properties of BU-4724V are summarized in Tables 5 and 6. Elemental analysis gave values of C, 51.34%; H, 5.85%, N, 13.74%, S, 5.78%. The IR spectrum of BU-4724V in potassium bromide is shown in Figure 1 and clearly indicates that BU-4724V is a peptide. The following peaks are evident: 3300, 2960, 1660, 1515, 1230 cm$^{-1}$.

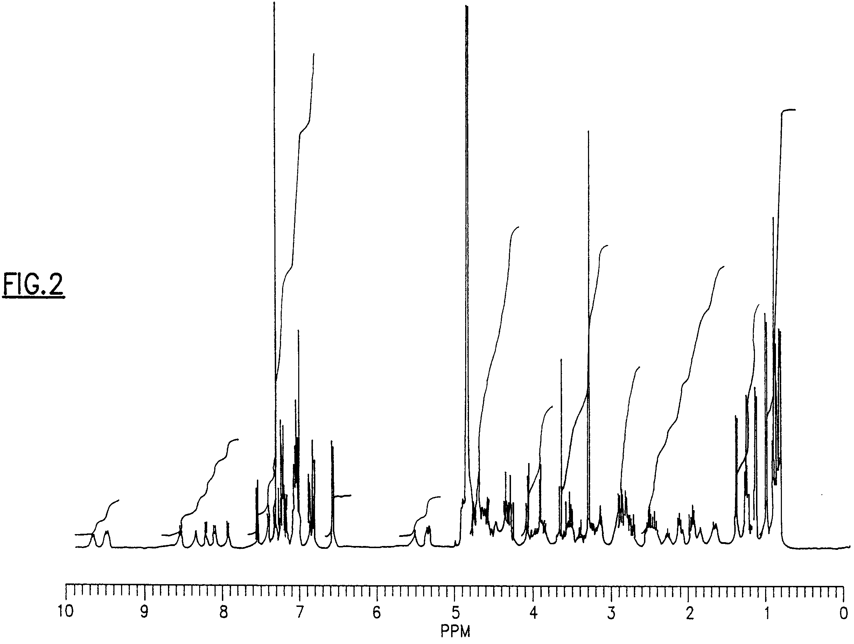

The $^1$H NMR spectrum of BU-4724V (400 MHz, $CD_3OD$) is shown in Figure 2 and the resonance patterns and chemical shifts are summarized in Table 6. The FAB-mass spectrum, elemental analysis and amino acid analysis indicate that BU-4724V has a molecular weight of 2162.

## TABLE 5

### Physico-Chemical Properties of BU-4724V

| | |
|---|---|
| Nature | white amporphous powder |
| MP | 255°C (dec) |
| $[\alpha]_D^{27°}$ (c 0.5, MeOH) | $-91 \pm 0.8°$ |
| Microanalysis Found | C: 51.34%; H: 5.85%; N: 13.74%; S: 5.78%; |
| UV $\lambda_{max}$ (MeOH) | 281 nm ($\epsilon$ 6,900) |
| IR $\nu$ (KBr) cm$^{-1}$ | 3300, 2960, 1660, 1515, 1230 |
| FAB-MS (m/z) | 2163 (M+H)$^+$ |
| HPLC* Rt. | 13.1 min. |
| TLC** Rf | 0.51 (n-BuOH-AcOH-H$_2$O = 4:1:1) 0.08 (CH$_2$Cl$_2$-MeOH-28% NH$_4$OH=16:4:1) |

---

\* Column: YMC A-301-3 (ODS, 4.6 mm i.d. x 100 mm, YMC Co.), Mobile phase: CH$_3$CN - 0.15% KH$_2$PO$_4$ (4:6) pH 3.5; Flow rate: 1.0 ml/min, Detection: UV at 214 nm

\*\* Silica gel Merck Art. 5715

**TABLE 6**

| ¹H NMR Spectral Data of BU-4724V (400 MHz, CD₃OD) | |
|---|---|
| 0.82 (3H, d, J=6.8 Hz) | 3.83-4.02 (4H, m) |
| 0.83 (3H, d, J=6.8 Hz) | 4.07-4.11 (2H, m) |
| 0.86 (3H, d, J=6.8 Hz) | 4.26-4.38 (4H, m) |
| 0.89 (3H, d, J=6.8 Hz) | 4.48 (2H, m) |
| 0.90 (3H, t, J=7.3 Hz) | 4.55-4.80 (8H, m) |
| 1.05 (3H, d, J=8.6 Hz) | 4.8-4.95 (overlap with HDO) |
| 1.07 (3H, d, J=8.6 Hz) | 5.35 (1H, dd, J=10.5, 3.9 Hz) |
| 1.13 (3H, d, J=6.8 Hz) | 5.52 (1H, t, J=10.5 Hz) |
| 1.18 (1H, m) | 6.57 (2H, d, J=8.6 Hz) |
| 1.24 (3H, d, J=6.8 Hz) | 6.83 (2H, d, J=8.6 Hz) |
| 1.37 (3H, d, J=6.8 Hz) | 6.88 (2H, d, J=6.4 Hz) |
| 1.64 (2H, m) | 7.00-7.11 (7H, m) |
| 1.83 (1H, m) | 7.15-7.34 (5H, m) |
| 1.93 (2H, m) | 7.42 (2H, s) |
| 2.10 (2H, m) | 7.56 (1H, d, J=7.7 Hz) |
| 2.26 (1H, m) | 7.93 (1H, br-d, J=10.4 Hz) |
| 2.38-2.54 (4H, m) | 8.11 (1H, d, J=9.4 Hz) |
| 2.67-2.97 (8H, m) | 8.34 (1H, br-d) |
| 3.12-3.28 (4H, m) | 8.56 (1H, d, J=9.0 Hz) |
| 3.32-3.43 (2H, m) | 9.49 (1H, d, J=9.0 Hz) |
| 3.49-3.68 (5H, m) | 9.66 (1H, br-d) |

The antiviral antibiotic BU-4724V was hydrolyzed for 48 hours in a sealed tube with 6N hydrochloric acid at 110°C under a nitrogen atmosphere, and the hydrolysate was subjected to amino acid analysis. Analysis showed the presence of the following amino acids; Asp(2), Ser(1), Gly(4), Ala(2), Tyr(1), Val(2), Ile(1), Leu(1) and Phe(2).

One mole of Trp was additionally detected by the hydrolysis of BU-4724V in the presence of 3M mercaptoethanesulfonic acid (100°C, 48 hours, in a sealed tube). In order to clarify Cys content, S-carboxymethylation of BU-4724V was performed with dithiothreitol followed by iodoacetic acid. Four moles of S-carboxymethyl-Cys were detected by the hydrolysis of S-carboxymethyl-BU-4724V (6N HCl, 110°C, 48 hours, in a sealed tube), which indicated the presence of four moles of Cys in BU-4724V.

From the hydrolysis experiments described above it was concluded that BU-4724V was constituted of 21 amino acids, Asp (2), Ser(1), Gly(4), Ala(2), Tyr(1), Val(2), Ile(1), Leu(1), Phe(2), Trp(1) and Cys(4). From an analysis of the data, it appears that BU-4724V may be a cyclic peptide.

The antiviral antibiotic designated BU-4724V has physico-chemical properties which in substantially pure form:

(a) is a white amorphous powder;
(b) has a melting point of 255°C (dec.);
(c) has a specific optical rotation

$$[\alpha]\,^{27}_{D}$$

of -91 ± 0.8° (c 0.5, $CH_3OH$);

(d) exhibits an infrared absorption spectrum (KBr) substantially as shown in Fig. 1;

(e) exhibits a 400 MHz proton magnetic resonance spectrum in $CD_3OD$ substantially as shown in Fig. 2;

(f) exhibits an ultraviolet absorption maxima ($\lambda_{max}$) in methanol of 281 nm ($\varepsilon$ 6,900);

(g) exhibits an elemental analysis for C, 51.34%; H, 5.85%; N, 13.74% and S, 5.78%;

(h) is soluble in dimethyl sulfoxide, dimethylformamide, methanol and ethanol, and is slightly soluble in alkaline water, and is insoluble in water, ethyl acetate, chloroform, benzene, n-hexane and petroleum ether;

(i) exhibits a positive color reaction to sulfuric acid and a negative color reaction to ninhydrin reagent;

(j) exhibits a molecular ion $(M+H)^+$ of 2163 as determined by FAB mass spectroscopy;

(k) has an Rf value of 0.51 in thin-layer chromatography using silica gel with n-BuOH-AcOH-$H_2O$ (4:1:1);

(l) exhibits the presence of amino acids after conventional acid hydrolysis with 6N HCl at 110°C for 48 hours in a sealed tube.

ANTIVIRAL ACTIVITY OF BU-4724V

1. Anti-HIV Activity

The antiviral antibiotic BU-4724V was evaluated for activity against human immunodeficiency virus (HIV-1 RF and HIV-1 IIIb strains were obtained from Robert Gallo, and HIV-2 CBL-20 strain obtained from Robin Weiss, all from the AIDS Research and Reference Program, Division of AIDS, NIAID, NIH) in CEM-SS cells (P.L. Nara et al. in AIDS Res. Human Retroviruses, 3, 283-302, 1987) using the XTT assay described by D.S. Weislow et al. in J. Natl. Cancer Institute, 81, 577-586, 1989. The CEM-SS cells were obtained from Peter Nara, AIDS Research and Reference Program, Division of AIDS, NIAID, NIH.

The antiviral effect is expressed as the inhibitory concentration of a compound which reduces the number of viruses of the infected culture to 50% of that of the drug - untreated, control culture ($IC_{50}$). The cytotoxicity is expressed as the concentration of a compound which yields 50% of the number of viable cells of the un-treated control cultures ($IC_{50}$). In this assay, 2',3'-Dideoxyinosine (ddI, Videx®) was also tested for anti-HIV activity. The results shown in Table 7 confirm that BU-4724V is active against three different strains of HIV and appears to be more active than ddI which has an $IC_{50}$ value of about 1-2 µM and, like ddI, showed very weak cytotoxicity against the test CEM-SS cells. The cytotoxicity of BU-4724V in CEM-SS cells was found to have an $IC_{50}$ value of about 150 µM.

**TABLE 7**

| Anti-HIV Activity of BU-4724V in CEM-SS Cells | |
| --- | --- |
| Strain | $IC_{50}$ µM |
| HIV-1 RF | 0.09-0.6 |
| HIV-1 IIIb | 0.46 |
| HIV-2 CBL-20 | 0.23 |

2. Anti-HSV Activity

The in vitro antiviral activity of BU-4724V was assessed against the herpes simplex virus type 1 (HSV-1) using the KOS strain. The assay was done according to the reported protocol of C. McLaren et al. in Antiviral Research, 3, 223-234, (1986) with minor modification. Briefly, 50 µl of Eagle's MEM medium containing BU-4724V and then 200 µl of the Vero cell suspension (2 X $10^4$ cells/200 µl) were added to a well of a 96-well microtiter plate. To the well, 50µl of medium containing approximately 30 times of the 50% tissue culture infectious dose (50% TCID) of virus was added. In a parallel experiment, the same set of cells without virus infection was prepared, which was for the determination of cytotoxicity of the sample. After 72 hours incubation at 37°C, the inhibition degree of virus-induced cytopathic effect and cytotoxicity by the sample were determined, respectively. The $ID_{50}$ is expressed as the concentration of a compound showing 50% growth inhibition of the cytopathic effect and the $TD_{50}$ as the concentration of a compound showing 50% growth inhibition of the cells in the absence of virus. Acyclovir was used as the reference compound for anti-HSV activity in the assay.

The results shown in Table 8 show that BU-4724V shows a weak but genuine anti-HSV activity as compared with acyclovir.

## TABLE 8

| Anti-HSV activity of BU-4724V and acyclovir | | |
|---|---|---|
| Compound | $ID_{50}$ (µg/ml) | $TD_{50}$ (µg/ml) |
| BU-4724V | 48 | >100 |
| Acyclovir | 0.28 | >100 |

3. Antibacterial Activity

The in vitro antibacterial activity of BU-4724V against various test organisms were determined by the general standard serial two-fold dilution method after overnight incubation at 32°C. As shown in Table 9, BU-4724V shows fairly good antibacterial activity against Gram-positive bacteria.

## TABLE 9

### Antibacterial activity of BU-4724V

| Test organism | | MIC (µg/ml) |
|---|---|---|
| S. aureus | FDA 209P JC-1 | 3.1 |
| " | Smith | 6.3 |
| " | A15036 (MRSA) | 3.1 |
| M. luteus | ATCC 9341 | 1.6 |
| B. subtilis | ATCC 6633 | 1.6 |
| E. coli | Juhl | >100 |
| " | K12 | >100 |
| " | NIHJ JC-2 | >100 |
| K. pneumoniae | ATCC 10031 | >100 |
| C. freundii | GN 7391 | >100 |
| S. typhi | 901 | >100 |
| P. aeruginosa | A9843A | >100 |

Medium: Nutrient Agar (Difco), pH 7.0
Inoculum size: $10^5$ cells/ml
Incubation conditions: 32°C, 18 hours

METHODS OF USE AND PHARMACEUTICAL COMPOSITIONS

The antiviral antibiotic BU-4724V, according to the present invention and pharmaceutical compositions thereof, are useful to inhibit the growth of Gram-positive bacteria and of viruses, including the human immunodeficiency and herpes simplex viruses. The antibacterial and antiviral effects of BU-4724V was demonstrated as described above.

The present invention, therefore, provides a method for therapeutically treating an animal host affected by a bacterial infection or by a retroviral infection which comprises administering to said host an effective antibacterial or antiviral-inhibiting dose of BU-4724V, or a pharmaceutical composition thereof.

In general, BU-4724V may be administered orally or parenterally in its pure-solid form, in dilute solution or suspension or in a concentrate and prepared for unit dose or multi-dose presentation. When administered parenterally, by intravenous or intramuscular or subcutaneous injection, or when administered orally, the dosage administered will be dependent on the age and weight of the mammalian species being treated, the route of administration, and the type and severity of the infectious condition being treated and other factors readily evaluated by the physician or veterinarian in attendance.

In respect to pharmaceutical compositions containing the antibiotic herein, carrier and other ingredients should be such as not to diminish the therapeutic effects of the antibiotic. Suitable dosage forms for oral use are tablets, dispersible powders, granules, capsules, syrups, and elixirs. Examples of parenteral forms are solutions, suspensions, dispersions, emulsions, and the like. The compositions for oral use may contain one or more conventional adjuvants, such as sweetening agents, flavoring agents, coloring agents, and preserving agents, in order to provide a composition of suitable pharmaceutical elegance. Tablets may contain the active ingredient in admixture with conventional pharmaceutically acceptable excipients, including inert diluents such as calcium carbonate, sodium carbonate, lactose, and talc; granulating and disintegrating agents such as starch and alginic acid; binding agents such as starch, gelatin, and acacia; and lubricating agents such as magnesium stearate, stearic acid, and talc. The tablets may be uncoated or coated by known techniques to delay disintegration and absorption in the gastrointestinal tract and thereby provide a sustained action over a longer period. Similarly, suspensions, syrups, and elixirs may contain the active ingredient in admixture with any of the conventional excipients utilized for the preparation of such compositions, such as suspending agents (e.g., methylcellulose, tragacanth, and sodium alginate), wetting agents (e.g., lecithin, polyoxyethylene stearate), and preservatives, such as ethyl p-hydroxybenzoate. Capsules may contain the active ingredient alone or admixed with an inert solid diluent, such as calcium carbonate, calcium phosphate, and kaolin. The injectable compositions are formulated as shown in the art and may contain appropriate dispersing or wetting agents and suspending agents identical or similar to those mentioned above.

The daily dosage for adult human treatment will preferably range from about 100 mg to about 2,000 mg of the active BU-4724V for a 70 kg adult, depending on the nature of the infection and the frequency and route of administration inter alia. It will be appreciated that in some instances, e.g., in the treatment of neonates, infants, and juveniles, smaller dosages than adult dosages may be desired.

The pharmaceutical compositions of this invention, thus, will generally comprise an effective antibacterial or antiviral-inhibiting amount of BU-4724V in combination with a suitable pharmaceutical acceptable carrier, such as water or alcohols which may contain fillers, stabilizers, wetting agents, emulsifying agents, and dispersing agents, to name but a few conventional carriers, adjuvants, and excipients which may be employed.

For use as an antibacterial agent, BU-4724V or a pharmaceutical composition thereof is administered so that the concentration of active ingredient is greater than the minimum inhibitory concentration for the particular organism being treated. For use as an antiviral agent, optimal dosages and regimes of BU-4724V for a given mammalian host can be readily ascertained by those skilled in the art. It will, of course, be appreciated that the actual dose of BU-4724V used will vary according to the particular composition formulated, the mode of application and the particular situs, host and disease treated. For use in the treatment of retroviral infections and, specifically, for the treatment of human immunodeficiency virus (HIV), it is contemplated that the treatment and dosage of BU-4724V would be similar to the treatment and dosage used with ddI (Videx®) and that the dosage would be adjusted accordingly by one skilled in the art to reflect the relative level of activity, for example, about 2 to about 5 times less than the relative dose of treatment with ddI. As will be appreciated by those skilled in the art many factors that modify the action of the drug will be taken into account including age, weight, sex, diet, time of administration, route of administration, rate of excretion, condition of the patient, drug combinations, reaction sensitivities and severity of the disease. Administration can be carried out continuously or periodically within the maximum tolerated dose. Optimal application rates for a given set of conditions can be ascertained by those skilled in the art using conventional dosage determination tests in view of the above guidelines.

The following example is given by way of illustration only and is not to be construed to limit the scope of the invention. Many apparent variations are possible without departing from the spirit and scope of the invention.

## SYNCYTIUM FORMATION INHIBITION ASSAY

The assay system for screening anti-syncytium formation agents consists of two cell lines, recombinant vaccinia virus-infected BSC-1 cells (gp-120 expressed) and HeLa-T4 cells (CD4 antigen expressed). Two cell lines were mixed together in the presence or absence of an inhibitor and the number of syncytia formed within $3 \sim 5$ hours was scored.

### Cultivation of assay cells

CD4-bearing HeLa (HeLa-T4) cells (P. J. Maddon, et al., *The $T_4$ gene encodes the AIDS virus receptor and* is expressed *in the immune system and the brain,* Cell, **47**, pp 333-348, 1986) were grown in Dulbecco modified Minimum Essential Medium (D-MEM, GIBCO) supplemented with 10% heat-inactivated fetal bovine serum (FBS, GIBCO) and 1 mg/ml geneticin (GIBCO). BSC-1 cells were grown in Eagle(E)-MEM (Nissui Pharmaceutical, Tokyo) supplemented with 10% FBS and 50 µg/ml amikacin (Bristol-Myers Squibb).

### Preparation of HIV gp-120 expressing BSC-1 cells

A monolayer culture of BSC-1 cells (3-day-old confluency) in a T-75 LUX flask (Sanko Junyaku, Tokyo) was inoculated by recombinant vaccinia virus (S. L. Hu, S. G. Kosowski, J. M. Dalrymple, *Expression of AIDS virus envelope gene in recombinant vaccinia viruses,* Nature, **320**, pp 537-540, 1986) at an MOI (Multiplicity of infection) of 0.01. After viral adsorption within one hour, the viral inoculum was removed by aspiration and the virus-infected cells were added 8 ml of E-MEM and incubated at 37°C for further 20 to 24 hours in a humidified 5% $CO_2$ and 95% air environment. After the infected cells were removed from the plastic flask surface using a cell scraper, the syncytium formation titer of the cell suspension, designated HIV gp-120 expressing BSC-1 cells, was determined to be $6 \times 10^4$ syncytia per milliliter by the 2-serial dilution method using HeLa-T4 cells as the test cells.

### Syncytium formation inhibition assay

A HeLa-$T_4$ cell suspension (100 µl containing $3 \times 10^4$ cells) was seeded in each well of the 96-well microtiterplate and grown at 37°C for 20 to 24 hours in a humidified 5% $CO_2$ and 95% air environment. The old medium was removed and replaced by 50 µl of the fresh E-MEM supplemented with 10% FBS which contained a test sample at various concentrations. Then the HIV gp-120 expressing BSC-1 cell suspension was diluted at 1:15. Fifty microliters of the dilution was added to each well of the 96-well microtiterplate. After incubation at 37°C for $3 \sim 5$ hours, the medium in each well was removed by aspiration. Cells were stained with 50 µl Giemsa solution (Wako Pure Chemicals, Osaka) and were washed 3 times with tap water. Number of syncytia in each well was scored under a microscope at a magnification of 40. Syncytium inhibitory activity of the compound is expressed in $ID_{50}$ (50% inhibitory dose) which is defined as the minimal concentration of the compound required to reduce syncytia formation by 50% as compared to the inhibitor-untreated control. Dextrane sulfate was used as a reference compound for syncytium formation inhibitory activity.

## EXAMPLE 1

## FERMENTATION OF BU-4724V

### A. Stocked culture

Streptomyces sp. AA6532 (ATCC 55290) was propagated on an agar slant composed of soluble starch (Nichiden Kagaku Co.) 0.5%, glucose 0.5%, meat extract (Mikuni Kagaku Kougyo Co.) 0.1%, yeast extract (Oliental Koubo Co.) 0.1%, NZ-case (Humko Sheffield Chemical Co.) 0.2%, NaCl 0.2%, $CaCO_3$ 0.1% and agar (Junsei Chemical Co.) 1.6%. After incubation at 28°C for 7 days, a portion of the mature agar slant was inoculated into 100 ml of a seed medium in a 500-ml Erlenmeyer flask and incubated for 4 days at 28°C on a rotary shaker (200 rpm). The seed medium was composed of soluble starch 2%, glucose 0.5%, NZ-case 0.3%, yeast extract 0.2%, fish meal D30X (Banyu nutrient) 0.5% and $CaCO_3$ 0.3%. The resulting vegetative mycelia were spun down (3,000 rpm, 15 min., 4°C) and resuspended with a half volume of 20% aq. glycerol solution and then stocked in -80°C.

B. Seed culture

A portion (0.3 ml) of the vegetative mycelia described above was inoculated into 100 ml of a medium in a 500-ml Erlenmeyer flask and incubated for 4 days at 28°C on a rotary shaker (200 rpm).

C. Flask fermentation

A 5-ml portion of the seed culture described above was transferred into 500-ml Erlenmeyer flasks each containing 100 ml of the production medium and incubated for 88 hours under the same conditions as those for the seed culture. The production medium consisted of lactose 2.0%, dextrin (Nichiden kagaku Co.) 2.0%, glucose 0.5%, cornsteep liquor (Oji Corn Starch Co.) 1.0%, rice bran 1.5%, $K_2HPO_4$ 0.05% and $CoCl_2 \cdot 6H_2O$ 0.0002%. The pH of the medium was adjusted to 7.0 before autoclaving. Antibiotic production in the fermentation broth was determined by Syncytium formation inhibitory activity.

EXAMPLE 2

ISOLATION AND PURIFICATION OF BU-4724V

The harvested fermentation broth (9.8 L) was extracted with n-butanol (5 L) and the extract was concentrated *in vacuo* to give a residual solid (13.9 g). The solid was applied on a column of Diaion HP-20 (Mitsubishi Chemical Industries Ltd., 40 mm i.d. x 300 mm), and the column was washed with 30% aqueous methanol (600 ml), then eluted with 80% aqueous methanol (800 ml). Evaporation of the eluate yielded a crude solid of BU-4724V (1.44 g). The crude BU-4724V was chromatographed on a column of YMC GEL ODS A60 (YMC Co., 40 mm i.d. x 450 mm) eluting with acetonitrile - 0.15% $KH_2PO_4$, pH 3.5 (4:6). The active fractions were pooled, concentrated and then extracted with n-butanol. Evaporation of the extract yielded white solid of semi-pure BU-4724V (703 mg). The solid was further purified on a column of Sephadex LH-20 (40 mm i.d. x 600 mm) eluted with methanol to yield white solid of pure BU-4724V (454 mg).

**Claims**

1. The antiviral antibiotic BU-4724V which in substantially pure form:
   (a) is a white amorphous powder;
   (b) has a melting point of 255°C (dec.);
   (c) has a specific optical rotation

$$[\alpha]^{27}_{D}$$

   of -91 ± 0.8° (c 0.5, $CH_3OH$);
   (d) exhibits an infrared absorption spectrum (KBr) substantially as shown in Fig. 1;
   (e) exhibits a 400 MHz proton magnetic resonance spectrum in $CD_3OD$ substantially as shown in Fig. 2;
   (f) exhibits an ultraviolet absorption maxima ($\lambda_{max}$) in methanol of 281 nm ($\varepsilon$ 6,900);
   (g) exhibits an elemental analysis for C, 51.34%; H, 5.85%; N, 13.74% and S, 5.78%;
   (h) is soluble in dimethyl sulfoxide, dimethylformamide, methanol and ethanol, and is slightly soluble in alkaline water, and is insoluble in water, ethyl acetate, chloroform, benzene, n-hexane and petroleum ether;
   (i) exhibits a positive color reaction to sulfuric acid and a negative color reaction to ninhydrin reagent;
   (j) exhibits a molecular ion $(M+H)^+$ of 2163 as determined by FAB mass spectroscopy;
   (k) has an Rf value of 0.51 in thin-layer chromatography using silica gel with n-BuOH-AcOH-$H_2O$ (4:1:1);
   (l) exhibits the presence of amino acids after conventional acid hydrolysis with 6N HCl at 110°C for 48 hours in a sealed tube.

2. A biologically pure culture of the microorganism Streptomyces sp. ATCC 55290, which is capable of producing the antiviral antibiotic BU-4724V in a recoverable quantity upon cultivation in a culture medium containing assimilable sources of carbon and nitrogen under submerged aerobic conditions.

3. The process for the preparation of BU-4724V, as defined in Claim 1, which comprises cultivating a BU-4724V-producing strain of Streptomyces sp. having the identifying characteristics of ATCC 55290, or a mutant thereof, in culture medium containing assimilable sources of carbon and nitrogen under submerged aerobic conditions until a substantial amount of BU-4724V is produced by said organism in said culture medium and then recovering BU-4724V from the culture medium.

4. A pharmaceutical composition comprising an effective retroviral-inhibiting amount of the antiviral antibiotic BU-4724V, as defined in Claim 1, and a pharmaceutically acceptable carrier or diluent.

5. Use of an effective retroviral-inhibiting amount of the antiviral antibiotic BU-4724V, as defined in claim 1, either alone or in a composition with a pharmaceutically acceptable carrier or diluent in the manufacture of a medicament for treating retroviral infection in a mammal.

6. Use of an effective retroviral-inhibiting amount of the antiviral antibiotic BU-4724V, as defined in claim 1, either alone or in a composition with a pharmaceutically acceptable carrier or diluent in the manufacture of a medicament for treating human blood cells infected with HIV, said medicament being administered to said cells.

WAVELENGTH IN MICRONS

FIG.1

WAVENUMBER (cm$^{-1}$)

PERCENT TRANSMISSION

EP 0 603 031 A2

FIG.2